# EUROPEAN PATENT APPLICATION

(11) **EP 0 837 094 A2**
(43) Date of publication of application: **22.04.1998**
(21) Application number: 97122750.9
(22) Date of filing: 23.03.1994
(51) Int. Cl.: C08K 5/098, C08L 27/06

(54) **Basic calcium carboxylates**

(30) Priority: 23.03.1993 GB 9305998
(62) Divisional of application: 94912499.4
(71) Applicant: EXXON CHEMICAL PATENTS INC., Linden New Jersey 07036 (US)
(72) Inventor: The designation of the inventor has not yet been filed
(74) Representative: Darby, David Thomas

(57) **Abstract**

Calcium superbase soaps are used as stabilisers for PVC.

## Description

The present invention relates to new basic calcium carboxylate compositions, a method for their preparation and their use in anticorrosion coatings particularly for cavity protection and underbody coating of automobiles to prevent chipping and corrosion, as lubricant additives and as polymer stabilisers.

Basic calcium carboxylates are known in many forms and are commonly used as additives in various lubricant systems and to impart anticorrosion properties to coatings such as temporary and permanent coatings for metals and as polymer stabilisers.

It is desirable to have as high a calcium level as possible in these products to reduce the amount of additive required to provide the desired calcium level. It has however proved difficult to develop a commercial process for the production of high calcium content calcium carboxylates prior to the use of various carboxylic acid mixtures as raw materials as described in our European Patent 0234149.

The process of European Patent 0234149 produces an oil solution of a high calcium content calcium carboxylate which is not useful in applications where the oil will act as a contaminant such as a plastics stabiliser. Furthermore the presence of the oil solvent increases the bulk and thus transportation costs.

Lower calcium content calcium salts such as calcium stearate have been used as polymer stabilisers particularly for polyvinyl chloride. The higher calcium content liquid calcium carboxylates such as those of our European Patent 0234149 cannot however be used since they have an adverse effect on the softening point of the polymer.

The traditional calcium salts however have a relatively low calcium content requiring substantial quantities of the product to achieve the desired effect.

There is therefore a need for a high solid content or 100% solid basic calcium carboxylate which may be incorporated into polymers, particularly as a stabiliser for polyvinyl chloride without an adverse effect of the viscosity of the coating medium to enable better handling.

We have now developed new solid calcium superbase soaps which do not possess the disadvantages of the known superbase soaps, which have a high TBN of the order of 400 or even higher, and which yield perfectly stable and clear solutions when employed.

The present invention therefore provides solid calcium superbase soaps in the form of calcium carbonate and calcium carboxylates, the carboxylic acids consisting of a mixture or otherwise of saturated organic carboxylic acids containing from 7 to 13 carbon atoms, having the following characteristics:
- a linear acid content of between 0 and 40% by weight,
- a content of acids which are branched on carbon 2 of between 0 and 20% by weight, and
- a content of acids which are mono- or polysubstituted on carbon 3 and/or on the carbons of higher rank, which is equal to or greater than 50% by weight.

The calcium superbase soaps are preferably prepared from saturated C₈, C₉ and C₁₀ organic carboxylic acids which consist of isomeric mixtures and which are generally known as oxo acids. These oxo acids are characterized by a low linear acid content, generally less than or equal to 10% by weight, a low content of acids which are branched on carbon 2, generally less than or equal to 10% by weight, and a high content of acids which are mono- or polysubstituted on carbon 3 and/or carbons of higher rank, which is generally greater than 80% by weight. The oxo acids are obtained by hydroformylation of C₇, C₈ and C₉ olefins, followed by an oxidation.

Still more preferably, the calcium soaps according to the invention are prepared from the C₈ saturated carboxylic acid (that is containing 8 carbon atoms) marketed under the trademark Cekanoic, this acid consisting of an isomeric mixture of octanoic acids containing at most 10% by weight of n-octanoic acid, at most 10% by weight of C₈ acids which are branched on carbon 2 and at least 80% by weight of C₈ acids which are branched on carbon 3 and/or the carbons of higher rank. In fact, it has surprisingly been found that the use of this acid makes it possible to obtain calcium soaps with a very high basicity of the order of 500 or higher.

Among the organic carboxylic acids which are also suitable for the present invention there may also be added the derivatives which are mono- or polysubstituted in the 3-position and/or of higher rank of the acids corresponding to heptanoic acid, octanoic acid, nonanoic acid, decanoic acid, undecanoic acid and dodecanoic acid. These include, for example, 3-methylhexanoic acid, isooctanoic acid, 4,5-dimethylhexanoic acid, isononanoic acid, 3,5,5-trimethylhexanoic acid, isodecanoic acid, 3-ethyloctanoic acid, isoundecanoic acid, 4-ethylnonanoic acid and isododecanoic acid. The mixture of one or more of the above-mentioned acids, whether mixed or not with their isomers, is also suitable for the present invention, it being understood that the content of linear acids does not exceed 40% and that the content of acids which are substituted on carbon 2 does not exceed 20%. We have found, in fact, that the linear acids and that the acids branched on carbon 2 lead to the formation of a viscous product, or to setting solid or, alternatively, to a precipitate which renders the product practically useless.

The present invention also relates to a process for the preparation of the calcium superbase soaps described above, according to which a calcium oxide and/or hydroxide is reacted, with stirring, with carbon dioxide (or CO₂) which is bubbled through the reaction mixture and at least one organic carboxylic acid, in the presence of at least one promoter which makes CO₂ fixation easier and at least one catalyst, and in that the water formed during the reaction is removed. The process according to the invention is characterized in that the reaction is performed in at least one organic solvent at a temperature of between 80 and 120_C, and in that the said acid is a saturated organic carboxylic acid containing from 7 to 13 carbon atoms, in which the content of linear acids is less than or equal to 40% by weight, in which the content of acids branched on carbon 2 is less than or equal to 20% by weight, and in which the content of acids branched on carbon 3 and/or the carbons of higher rank is equal to or higher than 40% by weight.

When the reaction has ended, after filtration, the organic solvent is removed by evaporation.

In accordance with a preferred embodiment of the process according to the invention, the organic solvent is allowed to evaporate during the reaction and is recycled into the reaction mixture so as to produce therein a bubbling action which promotes the reaction.

At least one nonpolar organic solvent chosen from naphtha, hexane, kerosene, benzene, toluene or xylene is used among the organic solvents which can be used in the process according to the invention. It is also possible to use a mixture of paraffinic hydrocarbons of mineral or synthetic origin, preferably containing a low proportion of aromatic and/or naphthenic hydrocarbons, such as white spirit. It is also possible to consider the use of polar organic solvents such as alcohols, for example 1-butanol, 2-butanol, ethylene glycol, propylene glycol, ethylene glycol monomethyl ether, ethylene glycol dimethyl ether, diethylene glycol and its ethers, mixtures of alcohols derived from paraffin, or methyl ethyl ketone.

The molar ratio of calcium to the organic carboxylic acid employed in the reaction is generally between 0.55 and 2, which corresponds to a basicity of between 1.1 and 4.

It should be recalled that the basicity is equal to the ratio of equivalents of calcium to the equivalents of carboxylic acids which are employed, that is to say, to the molar ratio of the calcium concentration to that of the carboxylic acids, multiplied by 2.

Furthermore, an hourly flow rate of carbon dioxide is imposed such that the hourly mass ratio of carbon dioxide to calcium is between 0.5 and 2, and preferably between 0.7 and 1.5.

Among the catalysts which may be used in the process according to the invention there may be mentioned metal oxides, for example zinc oxide, aluminium oxide Al₂O₃, silver oxide Ag₂O, magnesium oxide MgO, and zinc carboxylates such as zinc octanoate.

Among the promoters which make CO₂ fixation easier and which can be used in the present invention there may be mentioned labile hydrogen compounds such as alcohols, for example methanol, 2-propanol, octyl alcohol, ethylene glycol, triethylene glycol, stearyl alcohol, cyclohexylene glycol alcohol, cyclohexyl alcohol, aromatic alcohols such as phenol; amines, for example aniline, phenylenediamine, or dodecylamine; or, yet again, a mixture of alcohols and/or amines, for example of methanol and aqueous ammonia.

However, preferably, the material used is methanol, which gives the highest basicities and the shortest filtration times during the preparation of the calcium soap according to the invention.

In the calcium superbase soaps according to the invention, the promoters are used in a proportion of 1 to 25% by weight of final calcium salt, and preferably in a proportion of 5 to 15%.

The present invention will be better understood by reading the nonlimiting examples of the invention which follow.

A basic calcium salt was prepared by the process described in European Patent 0234149 with the additional step that the solvent is removed by heating the overbased carboxylate in an oven at 120°C and under a vacuum of 100 mn Hg until dry.

The solid calcium sulphonate was tested as an antiwear additive for the aliphatic ether lubricant which is predominantly C₂₀H₄₂O in the 4 Ball Wear Test (reference PSA D 55 1078) and the 4 Ball Extreme Pressure (EP) Test (reference PSA D 55 1136) with the following results.

| | **MD E20 Pure** | **MD E20 + 10% Calcium Carboxylate** | **MD E20 + 5% Calcium Carboxylate** |
|---|---|---|---|
| **4 Ball Wear Test** | | | |
| Scar Diam. (mm) | 0.455 | 0.34 | 0.37 |

| **4 Ball EP Test** | | | |
|---|---|---|---|
| Seizure Load (kg) | 45 | 80 | |
| Scar Diam. (mm) | 0.62 | 1.9 | |
| Load (kg)/Diam (mm) | 40/0.325 | 60/0.335 | |
| | 45/0.62 (seizure) | 70/0.36 | |
| | 50/1.711 | 75/0.39 80/1.9 (seizure) | |

## Claims

1. The use as a stabiliser for polyvinyl chloride of a solid calcium superbase soap having a basicity of 1.1 to less than 4, the soap being in the form of calcium carbonate and calcium carboxylates, the carboxylic acids consisting of a mixture or otherwise of saturated organic carboxylic acids containing from 7 to 13 carbon atoms, having the following characteristics:
* a linear acid content of between 0 and 40 % by weight,
* a content of acids which are branched on carbon 2 of between 0 and 20 % by weight, and
* a content of acids which are mono-or polysubstituted on carbon 3 and/or on the carbons of higher rank, which is equal to or greater than 50% by weight.

2. The use according to claim 1 in which the calcium superbase soap is prepared from saturated isomeric mixtures of C₈, C₉ and C₁₀ organic carboxylic acids.

3. The use according to claim 1 or 2 in which the acids are characterised by a low linear acid content, generally less than or equal to 10 % by weight, a low content of acids which are branched on carbon 2, generally less than or equal to 10 % by weight, and a high content of acids which are mono- or polysubstituted on carbon 3 and/or carbons of higher rank, which is generally greater that 80% by weight.

4. A stabilised polyvinyl chloride composition produced acording to claim 1, 2 or 3.
